# EUROPÄISCHE PATENTANMELDUNG

(11) **EP 2 100 583 A1**
(43) Veröffentlichungstag der Anmeldung: **16.09.2009**
(21) Anmeldenummer: 09151924.9
(22) Anmeldetag: 03.02.2009
(51) Int. Cl.: A61K 8/06, A61K 8/04, A61K 8/49, A61Q 1/02, A61K 8/40, A61K 8/895, A61K 8/891

(54) **Foundation mit partikulären organischen Lichtschutzfilter**

(30) Priorität: 10.03.2008 DE 102008013805
(71) Anmelder: Beiersdorf AG, 20253 Hamburg (DE)
(72) Erfinder: Riedel, Heidi, 22083, Hamburg (DE); Skubsch, Kerstin, 25421, Pinneberg (DE); Münchow, Lynn, 22547, Hamburg (DE)

(57) **Zusammenfassung**

Kosmetische Foundation die in Form einer Wasser-in-Silikonöl-Emulsion vorliegt, dadurch gekennzeichnet, dass die Zubereitung
a) mindestes einen partikulären organischen Lichtschutzfilter wie beispielhaft 2,2'-Methylen-bis-(6-(2H-benzotriazol-2-yl)-4-(1,1,3,3-tetramethylbutyl)-phenol) und/oder 2,4,6-Tris-(biphenyl)-1,3,5-triazin
b) einen oder mehrere weitere UV-Lichtschutzfilter gewählt aus der Gruppe der Verbindungen Dimethicodiethylbenzalmalonat (Polysilicone-15), 2-Ethylhexyl-2-cyano-3,3-diphenyl-acrylat (Octocrylen) und/oder 2-Phenylbenzimidazol-5-sulfonsäuresalze,
c) einen oder mehrere Farbstoffe und/oder Farbpigmente enthält.

## Beschreibung

Die vorliegende Erfindung betrifft eine kosmetische Foundation mit hohem Lichtschutzfaktor und hoher Deckkraft.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Das Gesichts-Make-up soll der Gesichtshaut ein natürliches Aussehen verleihen, blasse Haut auffrischen und farbliche Unregelmäßigkeiten der Haut ausgleichen.

Neben Gesichtspudern und Rouge als pulverförmigen Kosmetika, werden hierzu cremeförmige Präparate, wie Tagescremes und Creme-Make-up auf Emulsionsbasis verwendet. Eine besondere Ausführungsform ist die sogenannte Foundation. Als Foundation bezeichnet man dabei flüssige oder halbfeste Make-up-Präparate, die meist hautfarben sind (d.h. die Haut tönen) und auf das Gesicht, insbesondere auf die Wangen aufgetragen werden. Sie verleihen diesen ein gleichmäßiges gesundes gebräuntes oder rötliches Aussehen.

Foundations können in unterschiedlicher Form aufbewahrt, dargereicht und aufgetragen werden. Neben einfachen Flaschen und Tuben, Überdruck- oder Pumpspendern, kommen in jüngerer Zeit auch Flaschen und Tuben mit aufgesetztem Applikator, insbesondere Pinseln und Schwämmen zum Einsatz. Derartige Systeme haben den großen Vorteil, dass sich die Zubereitungen direkt aus dem Vorratsbehältnis auf die Haut auftragen lassen, ohne dass die Anwenderin sich in irgend einer Form "die Hände schmutzig machen" muss.

Nachteilig am Stande der Technik ist der Umstand, dass es schwierig ist, Foundations mit einem hohem Lichtschutzfaktor herzustellen. Größere Mengen an Lichtschutzfiltern führen in Foundations regelmäßig dazu, dass die Sensorik der Zubereitung darunter leidet. Die Foundations fühlen sich bei höheren Gehalten an UV-Filtern schmierig, fettig und/oder ölig an. Nach dem Auftragen auf die Haut bekommt dieselbe ein fettig-glänzendes Aussehen.

Ferner führen höhere Konzentrationen an Lichtschutzfiltern dazu, dass die Foundations an Deckkraft verlieren.

Nicht zuletzt kommt es bei größeren Gehalten an Lichtschutzfiltern zu Instabilitäten in der Zubereitung, d.h. zu Ölabscheidungen und ähnlichen Inhomogenitäten.

Es war daher die Aufgabe der vorliegenden Erfindung, die Mängel des Standes der Technik zu beseitigen und stabile sowie sensorisch attraktive Foundations zu entwickeln.

Überraschend gelöst werden die Aufgaben durch eine kosmetische Foundation die in Form einer Wasser-in-Silikonöl-Emulsion vorliegt, dadurch gekennzeichnet, dass die Zubereitung
a) mindestes einen partikulären organischen Lichtschutzfilter und
b) einen oder mehrere weitere UV-Lichtschutzfilter gewählt aus der Gruppe der Verbindungen Dimethicodiethylbenzalmalonat (Polysilicone-15), 2-Ethylhexyl-2-cyano-3,3-diphenylacrylat (Octocrylen) und/oder 2-Phenylbenzimidazol-5-sulfonsäuresalze,
c) einen oder mehrere Farbstoffe und/oder Farbpigmente enthält.

Die erfindungsgemäßen Zubereitungen sind einfach und kostengünstig herstellbar. Die Zubereitungen haben nach dem Auftragen auf die Haut eine hohe Deckkraft und ein gleichmäßiges aber dennoch natürliches, mattiertes Aussehen.

Nicht zuletzt weisen die erfindungsgemäßen Zubereitungen eine hohe dermatologische Verträglichkeit auf.

Zwar kennt der Fachmann die EP 1 709 954, WO 01/089466 und die WO 2005/065136, doch konnten diese Schriften nicht den Weg zur vorliegenden Erfindung weisen.

Erfindungsgemäß werden unter Wasser-in-Silikonöl-Emulsion Wasser-in-Öl-Emulsionen verstanden, deren Silikonölanteil in der Ölphase der Zubereitung mindestens 10 Gew.-% und bevorzugt mindestens 30 Gewichts % bezogen auf die Ölphase beträgt.

Es ist vorteilhaft im Sinne der vorliegenden Erfindung, wenn die Zubereitung partikuläre organische Lichtschutzfilter in einer Gesamtkonzentration von 0,1 bis 10 Gew.-%, bezogen auf das Gesamtgewicht der Zubereitung, enthält.

Es ist bevorzugt im Sinne der vorliegenden Erfindung, wenn die Zubereitung partikuläre organische Lichtschutzfilter in einer Gesamtkonzentration von 1 bis 7,5 Gew.-%, bezogen auf das Gesamtgewicht der Zubereitung, enthält.

Es ist erfindungsgemäß vorteilhaft, wenn als partikuläre organische Lichtschutzfilter a) 2,2'-Methylen-bis-(6-(2H-benzotriazol-2-yl)-4-(1,1,3,3-tetramethylbutyl)-phenol) und/oder 2,4,6-Tris-(biphenyl)-1,3,5-triazin eingesetzt werden.

Enthält die erfindungsgemäße Zubereitung 2,2'-Methylen-bis-(6-(2H-benzotriazol-2-yl)-4-(1,1,3,3-tetramethylbutyl)-phenol), so ist es erfindungsgemäß vorteilhaft, wenn die Zubereitung diese Verbindung in einer Menge von 0,5 bis 10 Gew.-% und bevorzugt in einer Menge von 1 bis 5 Gew.-%, jeweils bezogen auf das Gesamtgewicht der Zubereitung, enthält.

Enthält die erfindungsgemäße Zubereitung 2,4,6-Tris-(biphenyl)-1,3,5-triazin, so ist es erfindungsgemäß vorteilhaft, wenn die Zubereitung diese Verbindung in einer Menge von 0,5 bis 10 Gew.-% und bevorzugt in einer Menge von 1 bis 5 Gew.-%, jeweils bezogen auf das Gesamtgewicht der Zubereitung, enthält.

Vorteilhafte Ausführungsformen der vorliegenden Erfindung sind dadurch gekennzeichnet, dass die Farbstoffe und/oder Farbpigmente gewählt werden aus der Gruppe der Verbindungen mit dem Color-Index CI 77007, CI 77491, CI 77492, CI 77499, CI77891. Diese Stoffe werden erfindungsgemäß vorteilhaft in einer Gesamtmenge von 1- 20 Gew.-% und bevorzugt in einer Gesamtmenge von 5 -15 Gew.-%, bezogen auf das Gesamtgewicht der Zubereitung.

Erfindungsgemäß vorteilhaft ist es auch, wenn die erfindungsgemäße Zubereitung Farbstoffe und Farbpigmente in einer Gesamtkonzentration von 5 bis 15 Gew.-%, bezogen auf das Gesamtgewicht der Zubereitung, enthält.

Es kann erfindungsgemäß von Vorteil sein, wenn die erfindungsgemäße Zubereitung WeißPigmente gewählt aus der Gruppe der Verbindungen Kaolin, Titandioxid und Zinkoxid enthält.

In einem solchen Falle ist es erfindungsgemäß von Vorteil, wenn die Gesamtmenge an Weiß-Pigmenten von 2 bis 12 Gew.-%, bezogen auf das Gesamtgewicht der Zubereitung, beträgt.

Erfindungsgemäß vorteilhafte Ausführungsformen der vorliegenden Erfindung sind dadurch gekennzeichnet, dass die Zubereitung Dimethicodiethylbenzalmalonat (Polysilicone-15) enthält.

Es ist erfindungsgemäß vorteilhaft, wenn die Zubereitung Dimethicodiethylbenzalmalonat (Polysilicone-15) in einer Menge von 0,1 bis 10 Gew.-% und bevorzugt in einer Menge von 0,5 bis 5 Gew.-%, jeweils bezogen auf das Gesamtgewicht der Zubereitung, enthält.

Enthält die erfindungsgemäße Zubereitung 2-Ethylhexyl-2-cyano-3,3-diphenylacrylat (Octocrylen), so wird diese Komponente erfindungsgemäß vorteilhaft in einer Konzentration von 0,1 bis 10 Gew.-% und bevorzugt in einer Konzentration von 2,5 bis 7,5 Gew.-%, bezogen auf das Gesamtgewicht der Zubereitung eingesetzt.

Enthält die erfindungsgemäße Zubereitung 2-Phenylbenzimidazol-5-sulfonsäuresalze, so wird diese Komponente erfindungsgemäß vorteilhaft in einer Konzentration von 0,1 bis 10 Gew.-% und bevorzugt in einer Konzentration von 1 bis 5 Gew.-%, bezogen auf das Gesamtgewicht der Zubereitung eingesetzt.

Erfindungsgemäß werden unter 2-Phenylbenzimidazol-5-sulfonsäuresalze sowohl die 2-Phenylbenzimidazol-5-sulfonsäure (dissoziiert und undissoziiert) als auch ihre Salze verstanden.

Erfindungsgemäß bevorzugte Ausführungsformen der vorliegenden Erfindung sind dadurch gekennzeichnet, dass die Zubereitung
a) als partikulären organischen Lichtschutzfilter 2,2'-Methylen-bis-(6-(2H-benzotriazol-2-yl)-4-(1,1,3,3-tetramethylbutyl)-phenol) sowie
b) Dimethicodiethylbenzalmalonat (Polysilicone-15)
enthält.

Die erfindungsgemäße Zubereitung kann darüber hinaus noch weitere UV-Lichtschutzfiltersubstanzen enthalten.

Es ist vorteilhaft im Sinne der vorliegenden Erfindung, wenn die erfindungsgemäße Zubereitung einen Lichtschutzfaktor (SPF) von größer oder gleich 10 aufweist.

Es ist bevorzugt im Sinne der vorliegenden Erfindung, wenn die erfindungsgemäße Zubereitung einen Lichtschutzfaktor (SPF) von größer oder gleich 15 aufweist.

Erfindungsgemäß vorteilhaft wird der erfindungsgemäße SPF durch die erfindungsgemäße UV-Filterkombination aus 2,2'-Methylen-bis-(6-(2H-benzotriazol-2-yl)-4-(1,1,3,3-tetramethylbutyl)-phenol) und einem oder mehreren weiteren UV-Lichtschutzfilter gewählt aus der Gruppe der Verbindungen Dimethicodiethylbenzalmalonat (Polysilicone-15), 2-Ethylhexyl-2-cyano-3,3-di-phenylacrylat (Octocrylen) und/oder 2-Phenylbenzimidazol-5-sulfonsäuresalze bewirkt.

Erfindungsgemäß vorteilhafte Ausführungsformen der vorliegenden Erfindung sind dadurch gekennzeichnet, dass die Zubereitung einen Emulgator gewählt aus der Gruppe der Verbindungen PEG/PPG-19/19 dimethicon und Cetyl PEG/PPG-10/1 dimethicon enthält.

Enthält die erfindungsgemäße Foundation den Emulgator PEG/PPG-19/19 dimethicon, so wird dieser erfindungsgemäß vorteilhaft in einer Konzentration von 0,1 bis 10 Gew.-% und bevorzugt in einer Konzentration von 0,5 bis 5 Gew.-%, bezogen auf das Gesamtgewicht der Zubereitung eingesetzt.

Enthält die erfindungsgemäße Foundation den Emulgator Cetyl PEG/PPG-10/1 dimethicon, so wird dieser erfindungsgemäß vorteilhaft in einer Konzentration von 0,1 bis 10 Gew.-% und bevorzugt in einer Konzentration von 1 bis 7,5 Gew.-%, bezogen auf das Gesamtgewicht der Zubereitung eingesetzt.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind Permulen TR 1, TR 2, Carbopol 1328, Aristoflex AVC.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Die erfindungsgemäße Ölphase enthält erfindungsgemäß vorteilhaft Dimethicon und/oder Cyclomethicon und / oder Dimethiconol. Sie kann darüber hinaus auch noch weitere Öl- und/oder Wachskomponenten enthalten, beispielsweise Sheabutter, Myristyllactat, Propylencarbonat, Dicaprylylcarbonat, Octyldodecanol, Trimethylsiloxysilikat, Squalan, Sonnenblumenöl, Avocadoöl, Caprin / Capric Triglycerid, Kohlenwasserstoffe wie Isododecan, Isohexadecan, Polyisobuten, Paraffine.

Erfindungsgemäß vorteilhaft enthält die erfindungsgemäße Zubereitung einen oder mehrere Füll- und/oder Puderstoffe. Diese werden erfindungsgemäß vorteilhaft gewählt aus der Gruppe der Verbindungen Lauroyllysin, Talkum, Nylon, Silica, Methyl Methacrylate Crosspolymer, Reisstärke und/oder Polymethylsilsesquioxane.

Darüber hinaus enthält die erfindungsgemäße Zubereitung erfindungsgemäß vorteilhaft Stärkederivate wie Tapiocastärke, Distärkephosphat, Aluminium- bzw. Natrium-Stärke Octenylsuccinat.

Die erfindungsgemäße Zubereitung kann ferner einen oder mehrere der dem Fachmann bekannten kosmetischen Wirkstoffe enthalten, beispielsweise Tocopherylacetat.

Die erfindungsgemäße Foundation enthält erfindungsgemäß vorteilhaft einen oder mehrere Konservierungsstoffe welche bevorzugt gewählt werden aus der Gruppe der Verbindungen Methylparaben, Ethylparaben, Propylparaben, Butylparaben, Phenoxyethanol, Isobutylparaben, Diazolidinylharnstoff.

Nicht zuletzt ist es erfindungsgemäß vorteilhaft, wenn die erfindungsgemäße Zubereitung einen oder mehrere Stoffe gewählt aus der Liste der folgenden Verbindungen enthält: Silica, Xanthangummi Natriumchlorid, Disteardimoniumhectorit, Polymethylsilsequioxan quervernetztes Dimethicon, Methylmethacrylat, Styrol/Acrylat-Copolymer Polyacrylat 2,6-Di-*tert*-butyl-4-methylphenol (BHT).

Erfindungsgemäß vorteilhaft enthält die erfindungsgemäße Zubereitung ein oder mehrere Parfümstoffe.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### W/S-Foundations:

| | 1 | 2 | 3 | 4 |
|---|---|---|---|---|
| | Gew.-% | Gew.-% | Gew.-% | Gew.-% |
| Wasser | Ad 100 | Ad 100 | Ad 100 | Ad 100 |
| Glycerin | 7 | 5 | 5 | 10 |
| Cyclomethicon + PEG/PPG-19/19 Dimethicon | | 4 | 2 | |
| Cetyl PEG/PPG-10/1 Dimethicon | 4 | | | 5 |
| Propylene Carbonat | 0,05 | 0,06 | 0,06 | 0,03 |
| Disteardimonium Hectorite | 0,25 | 0,4 | 0,2 | 0,1 |
| Sodium Chloride | 2 | | 1 | 2 |
| Squalan | 0,5 | | 0,5 | |
| Dicaprylyl Carbonate | | 5 | | 5 |
| Cyclomethicone | 10 | 12 | 20 | 15 |
| Dicaprylyl Ether | 2 | | | |
| Caprylic/Capric Triglycerid | 2 | | | |
| Dimethicon | 2 | 3 | | 4 |
| Myristyl Laktat | | | 1 | |
| Lecithin | 1 | | 0,5 | |
| Dimethicon + Trimethylsiloxysilikat | | | 3,5 | 2 |
| Lauroyl Lysine | 5 | | 2,5 | 3 |
| Talkum | 2 | 3 | 0,5 | |
| Nylon 6/12 | | 3 | 2 | 4 |
| Silica | | 3 | 4 | 3 |
| Dimethicon + Dimethicon Crosspolymer | 6 | 4 | | |
| Methyl Methacrylat Crosspolymer | | | 1 | 2 |
| Polymethylsilsesquioxan | 2 | 1 | | |
| VP/VA Copolymer | 0,1 | | | 0,2 |
| VP/Hexadecen Copolymer | 0,1 | | 0,5 | |
| Titandioxid (CI 77891) | 6 | 3 | 3 | 8 |
| Farbpigmente (CI 77492 + CI 77491 + CI 77499) | 5 | 5 | 2 | 7 |
| Effektpigmente (z.B. beschichtetes Mica) | | 3 | 1 | |
| Octocrylen | 2,5 | 5 | 7,5 | 4 |
| Titandioxid | 1 | 2 | | |
| partikulärer organischer Lichtschutzfilter¹ | 2 | 4 | 5 | 1 |
| Dimethicodiethylbenzalmalonat (Polysilicone-15) | 0,5 | 2 | 4 | 2,5 |
| 2-Phenylbenzimidazol-5-sulfonsäuresalze | 1 | 5 | 2 | 3 |
| Sodium Ascorbyl Phosphate | | | 0,1 | 0,2 |
| Tocopheryl Acetate | 0,5 | 0,5 | 1 | 1,5 |
| Ubiquinon, Q10 | | 0,05 | | |
| Phenoxyethanol + Parabene | 1 | 0,7 | | |
| Tetrasodium Iminodisuccinat | | 0,2 | | |
| Diazolidinyl Urea | | | 0,3 | 0,3 |
| Parfum | 0,5 | 0,5 | 0,5 | 0,5 |

| | | | | |
|---|---|---|---|---|
| ¹ 2,2'-Methylen-bis-(6-(2H-benzotriazol-2-yl)-4-(1,1,3,3-tetramethylbutyl)-phenol) und/oder 2,4,6-Tris-(biphenyl)-1,3,5-triazin | | | | |

## Patentansprüche

1. Kosmetische Foundation die in Form einer Wasser-in-Silikonöl-Emulsion vorliegt, **dadurch gekennzeichnet, dass** die Zubereitung
a) mindestes einen partikulären organischen Lichtschutzfilter und
b) einen oder mehrere weitere UV-Lichtschutzfilter gewählt aus der Gruppe der Verbindungen Dimethicodiethylbenzalmalonat (Polysilicone-15), 2-Ethylhexyl-2-cyano-3,3-diphenylacrylat (Octocrylen) und/oder 2-Phenylbenzimidazol-5-sulfonsäuresalze,
c) einen oder mehrere Farbstoffe und/oder Farbpigmente
enthält.

2. Kosmetische Foundation nach Anspruch 1, **dadurch gekennzeichnet, dass** in der Zubereitung als partikuläre organische Lichtschutzfilter 2,2'-Methylen-bis-(6-(2H-benzotriazol-2-yl)-4-(1,1,3,3-tetramethylbutyl)-phenol) und/oder 2,4,6-Tris-(biphenyl)-1,3,5-triazin eingesetzt werden.

3. Kosmetische Foundation nach einem der vorhergehenden Ansprüche, **dadurch gekennzeichnet, dass** die Farbstoffe und/oder Farbpigmente gewählt werden aus der Gruppe der Verbindungen mit dem Color-Index CI 77007, CI 77491, CI 77492, CI 77499, CI77891.

4. Kosmetische Foundation nach einem der vorhergehenden Ansprüche, **dadurch gekennzeichnet, dass** die Zubereitung Dimethicodiethylbenzalmalonat (Polysilicone-15) enthält.

5. Kosmetische Foundation nach einem der vorhergehenden Ansprüche, **dadurch gekennzeichnet, dass** die Zubereitung einen Lichtschutzfaktor (SPF) von größer oder gleich 10 aufweist.

6. Kosmetische Foundation nach einem der vorhergehenden Ansprüche, **dadurch gekennzeichnet, dass** die Zubereitung einen Emulgator gewählt aus der Gruppe der Verbindungen PEG/PPG-19/19 dimethicon und Cetyl PEG/PPG-10/1 dimethicon enthält.

7. Kosmetische Foundation nach einem der vorhergehenden Ansprüche, **dadurch gekennzeichnet, dass** die Zubereitung
a) als partikulären organischen Lichtschutzfilter 2,2'-Methylen-bis-(6-(2H-benzotriazol-2-yl)-4-(1,1,3,3-tetramethylbutyl)-phenol) sowie
b) Dimethicodiethylbenzalmalonat (Polysilicone-15)
enthält.

8. Kosmetische Foundation nach einem der vorhergehenden Ansprüche, **dadurch gekennzeichnet, dass** die Zubereitung partikuläre organische Lichtschutzfilter in einer Gesamtkonzentration von 1 bis 10 Gew.-%, bezogen auf das Gesamtgewicht der Zubereitung, enthält.

9. Kosmetische Foundation nach einem der vorhergehenden Ansprüche, **dadurch gekennzeichnet, dass** die Zubereitung Farbstoffe und Farbpigmente in einer Gesamtkonzentration von 5 bis 15 Gew.-%, bezogen auf das Gesamtgewicht der Zubereitung, enthält.

10. Kosmetische Foundation nach einem der vorhergehenden Ansprüche, **dadurch gekennzeichnet, dass** die Zubereitung Dimethicodiethylbenzalmalonat (Polysilicone-15) in einer Gesamtkonzentration von 0,1 bis 10 Gew.-%, bezogen auf das Gesamtgewicht der Zubereitung, enthält.
